# DEMANDE DE BREVET EUROPEEN

(11) **EP 0 857 462 A1**
(43) Date de publication de la demande: **12.08.1998**
(21) Numéro de dépôt: 98420006.3
(22) Date de dépôt: 16.01.1998
(51) Int. Cl.: A61B 17/10, A61B 17/88

(54) **Support pour agrafe de contention ou d'ostéosynthèse**

(30) Priorité: 16.01.1997 FR 9700600
(71) Demandeur: Memometal Industries, 73220 Aiton (FR)
(72) Inventeur: Prandi, Bernard, 74210 Seythenex (FR)
(74) Mandataire: Myon, Gérard Jean-Pierre

(57) **Abrégé**

Support de transport et de pose (1) pour agrafe de contention ou d'ostéosynthèse (2), ladite agrafe étant pourvue d'au moins deux pattes (3, 4) sensiblement parallèles destinées à pénétrer dans un os (13), ledit support étant apte à supporter ladite agrafe en maintenant libres les extrémités (3a, 4a) desdites pattes de ladite agrafe, caractérisé en ce que ledit support comprend au moins une branche (7), fixe et rigide, définissant deux espaces (10, 11) dans lesquels peuvent être insérées lesdites pattes, sensiblement perpendiculairement à ladite branche.

Le support peut être muni de trois branches (6, 7, 8) ou d'une unique branche (7).

L'invention permet une mise en place précise d'une agrafe sans variation dimensionnelle de celle-ci.

## Description

L'invention a trait à un support de transport et de pose pour agrafe de contention ou d'ostéosynthèse.

Une agrafe de contention ou d'ostéosynthèse, qui peut éventuellement être réalisée dans un alliage à mémoire de forme, est utilisée pour maintenir en position relative deux parties d'un os en vue de leur consolidation.

Il est connu d'utiliser des agrafes en alliage de titane et de nickel, à mémoire de forme, dont la température de transition entre l'état martensitique et l'état austénitique est bien inférieure à la température normale du corps humain. Avant leur pose, ces agrafes sont conservées dans une enceinte réfrigérée, puis elles sont mises à la disposition du chirurgien dans le bloc opératoire. Or, au moment de la pose d'une agrafe, le chirurgien peut être confronté à une difficulté de nature à retarder les opérations de pose, au point que l'agrafe peut être portée à une température telle que sa forme est modifiée par transition martensitique/austénitique et qu'elle devient inutilisable. Il est alors nécessaire d'avoir recours à une autre agrafe.

Il est également connu d'utiliser des agrafes dites "chaudes" dont la température de transition est supérieure à celle du corps, par exemple de l'ordre de 55°C. Un risque de déformation existe pendant le transport ou le stockage de ces agrafes où la température de l'atmosphère ambiante peut atteindre, voire dépasser, 50°C

Pour pallier ces inconvénients, il est connu de la demande EP-A-0 682 920 d'utiliser un dispositif de conditionnement de l'agrafe en position d'utilisation, de sorte qu'elle est maintenue en forme y compris lorsque sa température augmente à cause de celle de l'atmosphère ambiante. Cependant, dès que le chirurgien ou l'opérateur retire l'agrafe du dispositif de conditionnement connu, si celle-ci est à une température relativement élevée, elle prend immédiatement une forme telle qu'elle n'est plus utilisable. En particulier, si l'écartement des extrémités des pattes de l'agrafe est réputé avoir une certaine valeur à froid et si des orifices de pré-impaction ont été ménagés sur l'os, il est possible, que lorsqu'on présente l'agrafe sur l'os, les extrémités des pattes soient plus rapprochées l'une de l'autre que la distance séparant les orifices de pré-impaction. Le chirurgien doit alors exercer sur les extrémités de l'agrafe un effort d'ouverture qui ne peut pas être quantifié précisément et qui risque d'écarter de façon trop importante les extrémités des pattes l'une de l'autre. Cette opération est de nature à retarder l'intervention chirurgicale, ce qui n'est pas sans conséquence pour le patient.

Par le brevet US-A-2,544,492, on connaît une pince pour agrafe dans laquelle une mâchoire, mobile grâce à une liaison vis-écrou, est manoeuvrée pour venir coincer une agrafe dans la tête de la pince. Cette pince est particulièrement complexe et ne peut pas être destinée au transport des agrafes, car il s'agit d'un matériel onéreux qui ne peut pas être immobilisé pour une agrafe pendant plusieurs semaines, voire plusieurs mois correspondant à son stockage. En outre, la mise en place de l'agrafe dans cette pince ne peut pas être automatisée.

L'invention vise à résoudre ces problèmes et à proposer un support pour agrafe d'ostéosynthèse permettant de maintenir une agrafe à la position exacte souhaitée et à permettre la pré-impaction de l'agrafe alors que celle-ci est en place sur son support.

Dans cet esprit, l'invention concerne un support de transport et de pose pour agrafe de contention d'ostéosynthèse, ladite agrafe étant pourvue d'au moins deux pattes sensiblement parallèles destinées à pénétrer dans un os, ledit support étant apte à supporter ladite agrafe en maintenant libres les extrémités desdites pattes de ladite agrafe, caractérisé en ce que ledit support comprend au moins une branche, fixe et rigide, définissant deux espaces dans lesquels peuvent être insérées lesdites pattes, sensiblement perpendiculairement à ladite branche.

Grâce à l'invention, les extrémités de l'agrafe peuvent être pré-impactées dans l'os alors que l'agrafe est en place sur son support et donc maintenue à sa dimension nominale et ce, quelle que soit la température ambiante. En outre, le support de l'invention, permet de régler un éventuel viseur de percée de pré-orifices d'impaction de l'agrafe aux cotes exactes de l'agrafe, en réglant les guides de ce viseur directement sur les extrémités des pattes de l'agrafe qui sont libres.

Selon un premier mode de réalisation de l'invention, le support comprend trois branches définissant entre elles les deux espaces dans lesquels peuvent être insérées les pattes de l'agrafe. Cet aspect de l'invention permet de réaliser de façon particulièrement simple la fonction recherchée avec l'invention.

Dans ce cas, la branche centrale du support a avantageusement une rigidité supérieure à celle des branches latérales. Cet aspect de l'invention permet d'utiliser la rigidité de la branche centrale pour maintenir l'agrafe dans la position souhaitée, alors que la souplesse des branches latérales est de nature à permettre le dégagement de l'agrafe par rapport à son support.

On peut également prévoir que les branches latérales sont aptes à exercer sur l'agrafe un effort de coincement sensiblement perpendiculaire aux pattes de l'agrafe. Cet aspect de l'invention permet un maintien efficace de l'agrafe sur son support, notamment pendant son transport.

Selon un second mode de réalisation de l'invention, le support comprend une unique branche, les espaces dans lesquels peuvent être insérées les pattes étant définis de part et d'autre de cette branche. Ce mode de réalisation particulièrement simple est d'un prix de revient attractif.

Selon un autre aspect de l'invention, applicable quel que soit le mode de réalisation considéré, la ou les branche(s) qui défini(ssen)t les espaces présente(nt) des largeurs étagées permettant la réception d'agrafes de largeurs différentes. Ainsi, un unique support peut être utilisé avec plusieurs agrafes d'une même gamme.

Selon un autre aspect avantageux de l'invention, l'épaisseur de la ou des branches du support est inférieure à la hauteur des pattes de l'agrafe. Cet aspect de l'invention permet que les extrémités des pattes de l'agrafe dépassent du support lorsque l'agrafe est en place sur celui-ci, ce qui autorise une pré-impaction de l'agrafe alors qu'elle est en place sur le support et garantit un positionnement adapté des extrémités de ses branches lors de cette pré-impaction.

Selon un autre aspect avantageux de l'invention, le support comprend une poignée conformée pour permettre une manipulation du support en fonction de la morphologie de l'os sur lequel l'agrafe est prévue d'être mise en place. On peut en particulier prévoir que le support comprend une cambrure entre la poignée et une zone de support de l'agrafe. Cette cambrure facilite la préhension du support.

Selon une autre variante de l'invention, on peut prévoir que le support comprend un décrochement entre la poignée et une zone de support de l'agrafe. Ce décrochement permet le logement de la partie corticale de l'os lors du début de l'impaction de l'agrafe.

Selon un autre aspect avantageux de l'invention, la ou les branches du support sont biseautées à leur extrémité, ce qui facilite leur dégagement par rapport à l'agrafe.

Selon un autre aspect avantageux de l'invention, la ou les branches sont pourvues de rainures pour le passage d'un foret de perçage ou pour le guidage d'un viseur de percée dans les espaces dans lesquels peuvent être insérées les pattes de l'agrafe. Ceci permet de percer l'os à la côte exacte de l'agrafe en se servant du support comme d'un guide.

L'invention sera mieux comprise et d'autres avantages de celle-ci apparaîtront plus clairement à la lumière de la description qui va suivre de quatre modes de réalisation d'un support conforme à son principe, donnée uniquement à titre d'exemple et faite en référence aux dessins annexés dans lesquels :
- la figure 1 est une vue en perspective de l'extrémité d'un support conforme à l'invention ;
- la figure 2 est une vue analogue à la figure 1 alors qu'une agrafe d'ostéosynthèse est en place sur le support de la figure 1 ;
- la figure 3 est une représentation schématique d'une première étape de la pose d'une agrafe d'ostéosynthèse grâce au support de l'invention, celui-ci étant représenté en coupe selon le plan III-III à la figure 2 ;
- la figure 4 est une vue analogue à la figure 3 lors d'une seconde étape de la pose de l'agrafe ;
- les figures 5 et 6 sont des vues de côté de supports conformes à un second et à un troisième modes de réalisation de l'invention et
- la figure 7 est une vue analogue à la figure 1 pour un support conforme à un quatrième mode de réalisation de l'invention.

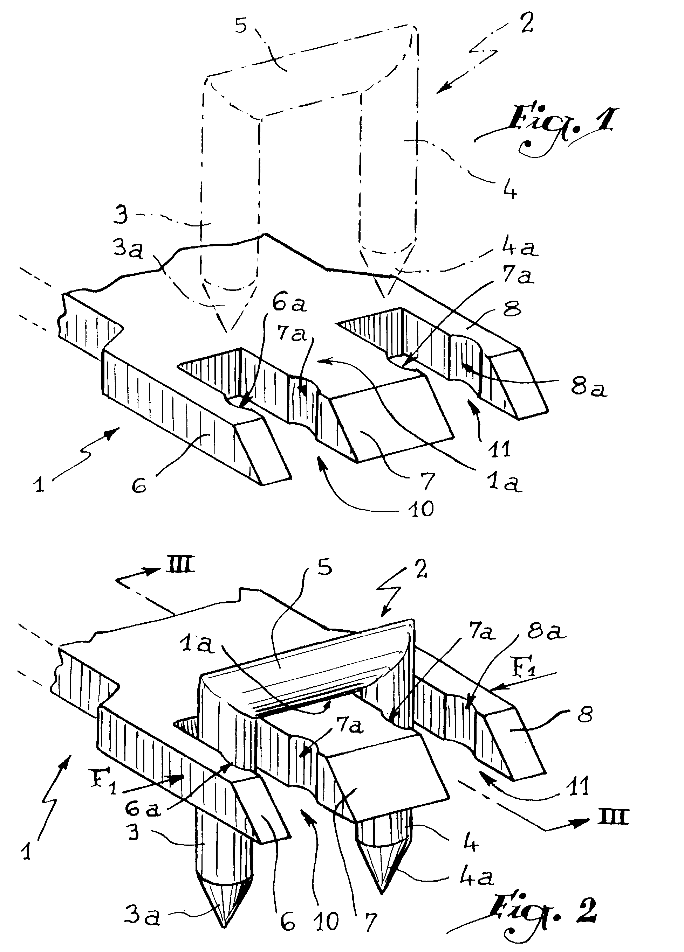

A la figure 1, on a représenté l'extrémité 1a d'un support 1 destiné au transport et à la pose d'une agrafe d'ostéosynthèse 2. L'agrafe 2 est pourvue de deux pattes 3 et 4 dont les extrémités 3a et 4a sont conformées en pointe afin de faciliter leur impaction dans un os. Les deux pattes 3 et 4 de l'agrafe 2 sont reliées par une partie transversale 5 globalement perpendiculaire aux pattes 3 et 4 qui sont sensiblement parallèles. La géométrie précise de l'agrafe 2 peut varier en fonction des choix du praticien et/ou du fabricant d'agrafe. Il peut en particulier s'agir d'une agrafe formée d'une bande de métal pliée.

Le support 1 comprend trois branches respectivement référencées 6, 7 et 8 qui s'étendent parallèlement à la surface principale du support 1 et définissent entre elles deux espaces 10 et 11 dans lesquels peuvent être insérées les pattes 3 et 4 de l'agrafe 2, sensiblement perpendiculairement aux branches 6 à 8. Le support 1 et l'agrafe 2 sont alors dans la position de la figure 2 dans laquelle l'agrafe est en chevauchement de la branche 7 et les extrémités 3a et 4a des pattes 3 et 4 sont libres, c'est-à-dire dépassent du support 1 du côté opposé à la partie transversale 5 de l'agrafe 2.

Dans la position de la figure 2, l'agrafe 2 est maintenue en position, de sorte qu'elle présente une bonne stabilité dimensionnelle quelle que soit la température ambiante. Ceci est particulièrement avantageux dans le cas d'une agrafe d'ostéosynthèse réalisée dans un alliage de titane et de nickel à mémoire de forme dont la température de transition est faible. En effet, même si l'agrafe est maintenue pendant une longue période dans une atmosphère ambiante à une température relativement élevée telle que celle régnant habituellement dans un bloc opératoire, elle ne peut pas se déformer dans la mesure où la branche centrale 7 empêche le rapprochement des extrémités 3a et 4a des pattes 3 et 4. L'invention est aussi utilisable avec une agrafe réalisée dans un matériau ne présentant pas de propriétés spécifiques de mémoire de forme.

On note que, selon un aspect avantageux de l'invention, la branche 7 est plus large que les branches 6 et 8, ce qui lui permet d'avoir une bonne rigidité. En fait, la branche 7 a une rigidité supérieure à celle des branches 6 et 8 car elle doit pouvoir résister à un effort de compression ou de torsion exercé par les pattes 3 et 4, en particulier lors d'un réchauffement de l'agrafe si celle-ci est réalisée en alliage de mémoire de forme.

On peut également prévoir que les branches 6 et 8 sont prévues pour exercer sur les pattes 3 et 4 de l'agrafe 2 un effort de coincement, représenté par les flèches F₁ à la figure 2, sensiblement perpendiculaire aux pattes 3 et 4. Cet effort F₁ peut être obtenu grâce à la géométrie des branches 6 et 7 ou au fait que les entailles 10 et 11 sont de dimensions légèrement inférieures au diamètre des pattes 3 et 4.

Les extrémités des pattes 6 à 8 sont biseautées, ce qui facilite leur dégagement et, par voie de conséquence, l'impaction de l'agrafe.

Les bords en regard des branches 6 à 8 présentent, en avant de l'agrafe 2 lorsqu'elle est en position comme représenté à la figure 2, des rainures 6a, 7a et 8a permettant le passage d'un foret de perçage dans les espaces 10 et 11. Il serait également possible de prévoir que les rainures sont situées en arrière de l'agrafe 2 lorsqu'elle est en place sur le support 1.

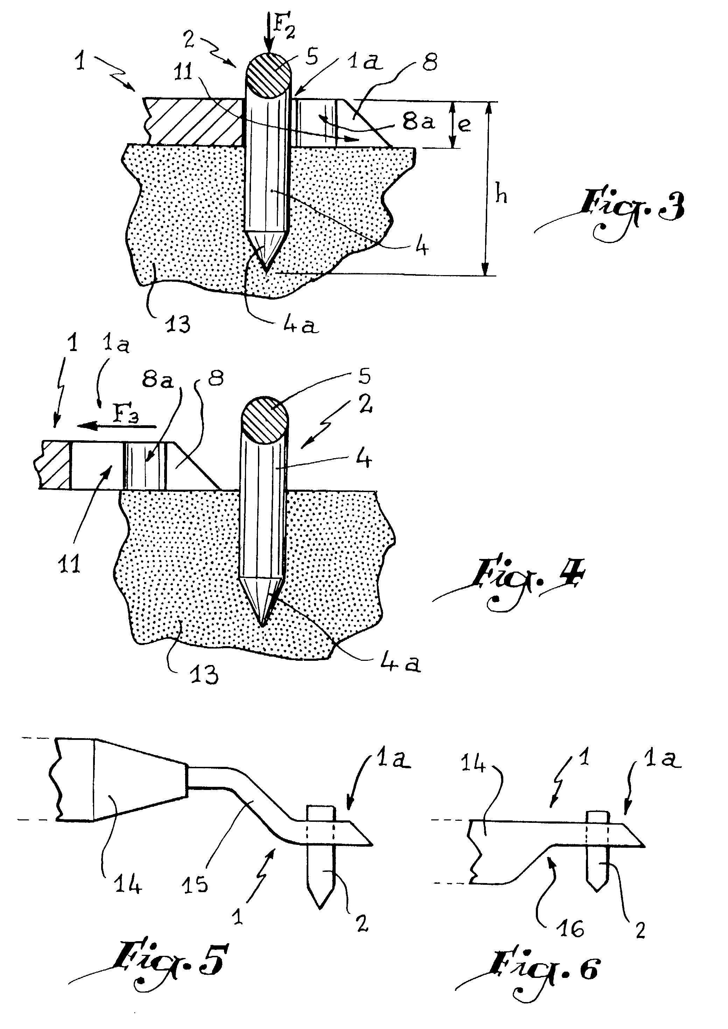

Comme il apparaît en particulier à la figure 3, l'épaisseur e des branches 6 à 8 est inférieure à la hauteur h des pattes 3 et 4. Ceci permet au support 1 de maintenir libres les extrémités 3a et 4a des pattes 3 et 4.

La pose d'une agrafe d'ostéosynthèse grâce au support de l'invention est effectuée comme suit :
i - On présente l'agrafe d'ostéosynthèse sur l'os, de telle sorte que les extrémités 3a et 4a de chacune de ses branches 3 et 4 soit respectivement en regard de deux parties d'un os 13 à maintenir rapprochées.
ii - On exerce sur l'agrafe 2 un effort F₂ d'impaction parallèle aux pattes 3 et 4, de telle sorte que celles-ci pénètrent dans l'os jusqu'à ce que le support vienne au contact de l'os 13 alors que la partie transversale 5 de l'agrafe 2 est en appui sur la branche 7 du support 1. On est alors dans la position de figure 3.
iii - En exerçant un effort F₃ de dégagement, et en maintenant l'agrafe en place grâce à un effort F₄ exercé sur sa partie 5, il est possible de retirer le support 1 alors que l'agrafe 2 est déjà, pour l'essentiel, en place dans l'os 13. Ceci est représenté à la figure 4.
iv - Il suffit alors de terminer l'impaction de l'agrafe 2 en répétant un effort analogue à l'effort F₂ de la figure 3.

Grâce au support de l'invention, l'agrafe peut donc être mise en place en étant en permanence maintenue à sa dimension nominale, de sorte qu'aucune imprécision ne résulte de l'utilisation d'une agrafe d'ostéosynthèse réalisée dans un alliage à mémoire de forme.

Lorsqu'il n'est pas possible ou souhaitable d'impacter directement l'agrafe dans l'os 13, par exemple lorsqu'il s'agit d'un os dur ou friable, on peut utiliser un viseur de percée qui permet de réaliser des pré-orifices d'impaction dans l'os 13. Dans ce cas, les guides du viseur de percée peuvent être réglés sur les extrémités 3a ou 4a de l'agrafe 2 qui sont libres, c'est-à-dire qui dépassent du support 1. Il est également possible de centrer le viseur de percée grâce aux rainures 6a, 7a et 8a des branches 6 à 8. Là encore, l'invention permet d'améliorer la précision obtenue lors de la pose d'une agrafe de contention ou d'ostéosynthèse.

Comme il apparaît plus clairement à la figure 5 représentant un second mode de réalisation d'un support conforme à l'invention, le support 1 peut comprendre une poignée 14, reliée à la zone 1a de support de l'agrafe 2 par une cambrure 15, de sorte que la poignée facilite la manipulation du support 1 en étant adaptée à la morphologie de l'os, non représenté, sur lequel l'agrafe doit être mise en place.

Dans le troisième mode de réalisation de l'invention représenté à la figure 6, le support 1 comprend un décrochement 16 entre une zone 1a de support d'une agrafe d'ostéosynthèse 2 et une poignée 14 de préhension du support 1. Ce décrochement permet le logement de l'os devant recevoir l'agrafe.

Selon une variante non représentée de l'invention, on peut également prévoir que les branches 6, 7 et 8 sont reliées par des bandes de matière sécable, ce qui évite tout risque de glissement de l'agrafe 2 hors des espaces 10 et 11. Ces bandes de matière sont déchiquetées lors du dégagement du support 1 représenté à la figure 4. D'autres modifications de forme du support 1 sont aussi envisageables.

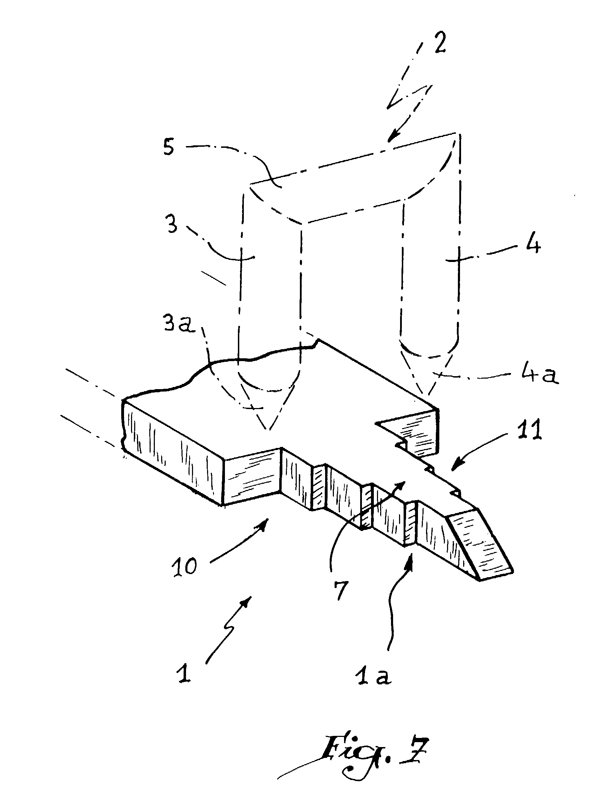

Dans le quatrième mode de réalisation de l'invention représenté à la figure 7, les éléments analogues à ceux du mode de réalisation des figures 1 à 4 portent des références identiques. Ce mode de réalisation diffère des précédents essentiellement en ce que le support 1 comprend une unique branche 7, fixe et rigide, autour de laquelle peut être disposée l'agrafe 2 de telle sorte que ces pattes 3 et 4 soient placées dans des espaces 10 et 11 définis de part et d'autre de la patte 7. Le support conforme à ce mode de réalisation est donc particulièrement simple à réaliser et de prix de revient attractif.

Conformément à un aspect avantageux également applicable aux modes de réalisation précédents, la branche 7 a deux faces latérales en escalier 7b de sorte qu'elle présente des largeurs étagées permettant de recevoir des agrafes de largeurs différentes. Ainsi, un seul support peut être utilisé avec différents types d'agrafes.

Comme précédemment, l'extrémité avant de la branche 7 est biseautée et son épaisseur est inférieure à la hauteur des pattes 3 et 4.

Quel que soit le mode de réalisation considéré, le support peut être réalisé dans tout type de matériau biocompatible et, notamment, en matière plastique ou en acier inox. Le support peut même être réalisé en matériau non bio-compatible si l'on peut assurer qu'aucun copeau ne peut être arraché.

Le support 1 peut servir au transport de l'agrafe 2 en étant inséré dans une enveloppe de protection telle que notamment, une boîte ou un blister, cette enveloppe étant ouverte au moment de l'utilisation de l'agrafe.

L'invention est utilisable quel que soit le type de l'agrafe employée. Il peut notamment s'agir d'une agrafe dite "froide" ou dite "chaude", d'une agrafe à profil en S ou en oeil, à deux, trois ou quatre pattes ou d'une lame agrafe utilisée, notamment, lors d'une intervention chirurgicale sur un genou.

## Revendications

1. Support de transport et de pose (1) pour agrafe de contention ou d'ostéosynthèse (2), ladite agrafe étant pourvue d'au moins deux pattes (3, 4) sensiblement parallèles destinées à pénétrer dans un os (13), ledit support étant apte à supporter ladite agrafe en maintenant libres les extrémités (3a, 4a) desdites pattes de ladite agrafe, caractérisé en ce que ledit support comprend au moins une branche (7), fixe et rigide, définissant deux espaces (10, 11) dans lesquels peuvent être insérées lesdites pattes, sensiblement perpendiculairement à ladite branche.

2. Support pour agrafe d'ostéosynthèse selon la revendication 1, caractérisé en ce qu'il comprend trois branches (6, 7, 8) définissant entre elles lesdits deux espaces (10, 11).

3. Support pour agrafe d'ostéosynthèse selon la revendication 2, caractérisé en ce que la branche centrale (7) dudit support (1) a une rigidité supérieure à celles des branches -latérales (6, 8) dudit support.

4. Support pour agrafe d'ostéosynthèse selon la revendications 2, caractérisé en ce que les branches latérales (6, 8) sont aptes à exercer sur ladite agrafe (2) un effort de coincement (F₁) sensiblement perpendiculaire auxdites pattes (3, 4) de ladite agrafe.

5. Support pour agrafe selon la revendication 1, caractérisé en ce qu'il comprend une unique branche (7), lesdits espaces (10, 11) dans lesquels peuvent être insérées lesdites pattes (3, 4) étant définis de part et d'autre de ladite branche.

6. Support pour agrafe selon l'une des revendications précédentes, caractérisé en ce que ladite ou lesdites branche (7) présente(nt) des largeurs étagées (7b) permettant la réception d'agrafes de largeur différentes.

7. Support pour agrafe selon l'une des revendications précédentes, caractérisé en ce que l'épaisseur (e) de ladite ou desdites branches (6, 7, 8) est inférieure à la hauteur (h) desdites pattes (3, 4) de ladite agrafe (2).

8. Support pour agrafe selon l'une des revendications précédentes, caractérisé en ce qu'il comprend une poignée (14) conformée pour permettre une manipulation dudit support en fonction de la morphologie de l'os sur lequel ladite agrafe (2) est prévue d'être mise en place.

9. Support pour agrafe selon la revendication 8, caractérisé en ce qu'il comprend une cambrure (15) entre ladite poignée (14) et une zone (1a) de support de ladite agrafe.

10. Support pour agrafe selon la revendication 8, caractérisé en ce qu'il comprend un décrochement (16) entre ladite poignée (14) et une zone (1a) de support de ladite agrafe.

11. Support pour agrafe selon l'une des revendications précédentes, caractérisé en ce que ladite ou lesdites branches (6, 7, 8) sont biseautées à leur extrémité.

12. Support pour agrafe selon l'une des revendications précédentes, caractérisé en ce que ladite ou lesdites branches (6, 7, 8) sont pourvues de rainures (6a, 7a, 8a) pour le passage d'un foret de perçage ou pour le guidage d'un viseur de percée dans lesdits deux espaces (10, 11).
